# EUROPEAN PATENT SPECIFICATION

(11) **EP 2 559 457 B1**
(45) Date of publication and mention of the grant of the patent: **17.10.2018**
(21) Application number: 11192539.2
(22) Date of filing: 24.01.2008
(51) Int. Cl.: A61Q 15/00, A61K 8/34, A61K 8/92, A61K 8/04, A61K 8/58, A61K 8/02

(54) **Antiperspirant/deodorant compositions**
Schweisshemmende/desodorierende Zusammensetzungen
Compositions anti-transpirantes/déodorantes

(30) Priority: 02.02.2007 US 670481; 11.01.2008 US 972882
(43) Date of publication of application: 20.02.2013
(62) Divisional of application: 08795788.2
(73) Proprietor: Colgate-Palmolive Company, New York, NY 10022 (US)
(72) Inventor: Misner, Steven H., Verona, NJ New Jersey 07044 (US); Broadwell, Roger, Dover, NJ New Jersey 07801 (US); Van Duyne, John, Jr., Rockaway, NJ New Jersey 07866 (US); Fan, Aixing, Bridgewater, NJ New Jersey 08807 (US); Jogun, Suzanne, Wayne, NJ New Jersey 07470 (US); Alfone, Stacy, Hillsborough, NJ New Jersey 08844 (US); Bertino, Mary, Cranford, NJ New Jersey 07016 (US); Cotero, Fernando, Pinehurst, NC North Carolina 28374 (US); Adams, Peter Richard, South Orange, NJ 07079 (US); Linn, Elizabeth, Lyndhurst, NJ New Jersey 07071 (US); Dharia, Hemani, Jersey City, NJ New Jersey 07307 (US)
(74) Representative: Wichmann, Hendrik

(56) References cited:
- EP-A- 1 529 521
- WO-A-99/11233
- US-A1- 2003 207 971
- US-A1- 2005 281 851
- DATABASE EPODOC EUROPEAN PATENT OFFICE, THE HAGUE, NL; JP62111912 22 May 1987 (1987-05-22), "Antiperspirant stick", XP002494545,

## Description

This relates to antiperspirant/deodorant compositions.

### BACKGROUND OF THE INVENTION

The majority of anhydrous antiperspirant/deodorant compositions contain a stearyl alcohol or n-alkane as a primary gellant and to provide a stable composition matrix. Alternative gellant options, most notably triglycerides from plant and animal tissues, have been explored and found to result in significant formulation changes, at increased cost. Use of these triglycerides in the composition often does not provide a desirable application or cosmetic aesthetic, providing a less structurally stable composition that leaves a visible residue.

It would be desirable to include a hydrogenated soybean oil into an antiperspirant/deodorant composition to provide a similar or improved structure over existing compositions or improve the aesthetics of the composition.

### SUMMARY OF THE INVENTION

The present invention provides a composition according to claim 1. Preferred features are defined in the dependent claims.

Also disclosed herein is a method comprising applying the composition of the invention to an axillary area.

Also disclosed herein is a method for increasing the fragrance retention of a composition comprising adding at least one soybean oil having an iodine value of 1 to 5 to the composition in an amount of up to 10% by weight of the composition, wherein the composition comprises:
i) at least one active chosen from at least one antiperspirant active and at least one deodorant active;
ii) at least one fatty alcohol; and
iii) at least one silicone.

### BRIEF DESCRIPTION OF THE DRAWINGS

Figure 1 is a Differential Scanning Calorimetry (DSC) graph of a composition containing stearyl alcohol as a gellant along with hydrogenated soybean oil as a co-gellant as compared to a composition containing stearyl alcohol as a gellant along with hydrogenated castor oil as a co-gellant.
Figure 2 is a Differential Scanning Calorimetry (DSC) graph of a composition containing polyethylene as a gellant along with hydrogenated soybean oil as a co-gellant as compared to a composition containing polyethylene as a gellant along with hydrogenated castor oil as a co-gellant.

### DETAILED DESCRIPTION OF THE INVENTION

As used throughout, ranges are used as a shorthand for describing each and every value that is within the range. Any value within the range can be selected as the terminus of the range.

The composition is a solid stick or soft solid when at ambient room temperature of about 25°C. The stick form is an example of a solid form, and the soft solid is a thickened form that may or may not be solid. The stick form can be distinguished from a soft solid in that, in a stick, the formulated product can retain its shape for extended time periods outside the package, the product not loosing its shape significantly (allowing for some shrinkage due to solvent evaporation). Adjustment of amounts of gelling or thickening agents can be used in order to form a soft solid or stick.

Soft solids can be suitably packaged in containers that have the appearance of a stick, but which dispense through apertures (for example, slots or pores) on the top surface of the package. The soft solid products have also been called soft sticks or "smooth-ons", and hereinafter are generically called "soft solids". Reference is made to U.S. Pat. No. 5,102,656, U.S. Pat. No. 5,069,897, and U.S. Pat. No. 4,937,069, each of which discloses such soft solids, including physical characteristics thereof such as viscosity and hardness.

### Gelling Agents

Gelling agents used in the present invention comprise hydrogenated soybean oil and a first gellant comprising a fatty alcohol and optionally a hydrocarbon of the formula CₙH₂ₙ₊₂, wherein n is about 20 to about 100, and the hydrocarbon is at least 90% linear.

The hydrogenated soybean oil is used as a co-gellant along with the first gellant to provide a solid stick or soft solid antiperspirant. The hydrogenated soybean oil is almost, but not fully hydrogenated. The amount of hydrogenation is measured by the iodine value. The iodine value can be measured by ASTM D5554-95 (2006). The iodine value of the hydrogenated soybean oil used herein is 1 to 5. It has been found that this level of hydrogenation provides the desired structure to the antiperspirant and provides a softer and creamier application aesthetics.

The hydrogenated soybean oil is present in an amount of 3 to 7% by weight of the composition. In one embodiment, the amount is about 4 to about 6% by weight.

The hydrogenated soybean oil can provide increased fragrance longevity when used to replace hydrogenated castor oil.

The fatty alcohol can be any fatty alcohol. In one embodiment, the fatty alcohol is stearyl alcohol.

The hydrocarbon is a hydrocarbon of the formula CₙH₂ₙ₊₂, wherein n is 20-100, and the hydrocarbon is at least 90% linear. In one embodiment, the hydrocarbon is a paraffin. In another embodiment, the hydrocarbon is polyethylene. An example of a polyethylene can be found in United States Patent No. 6,503,491. In another embodiment, the polyethylene has a weight average molecular weight in of about 300 to about 3000 and a melting point of about 50 to about 129°C.

In one embodiment, the first gellant is present in the composition in an amount of 5 to 25% by weight of the composition. In another embodiment, the amount is 10 to 20% by weight.

The formulations of the invention may further comprise additional gelling agents, which include, but are not limited to, waxes, esters of fatty acid and fatty alcohol, triglycerides, or other cosmetically acceptable materials, which are solid or semi solid at room temperature and provide a consistency suitable for application to the skin.

When the hydrogenated soybean oil is used in combination with the fatty alcohol, the resulting structure crystallizes at lower temperature. The following samples were prepared to show this effect.

| | A | B (control) |
|---|---|---|
| Cyclomethicone | 35.6 | 35.6 |
| Stearyl alcohol | 17 | 17 |
| C12-15 alkyl benzoate | 10 | 10 |
| PPG14 butyl ether | 5 | 5 |
| CASTORWAX™ MP80 | 2.8 | 7.4 |
| Hydrogenated soybean oil | 4.6 | 0 |
| PEG-8 distearate | 3 | 3 |
| Talc | 2 | 2 |
| Antiperspirant AZP908 | 20 | 20 |

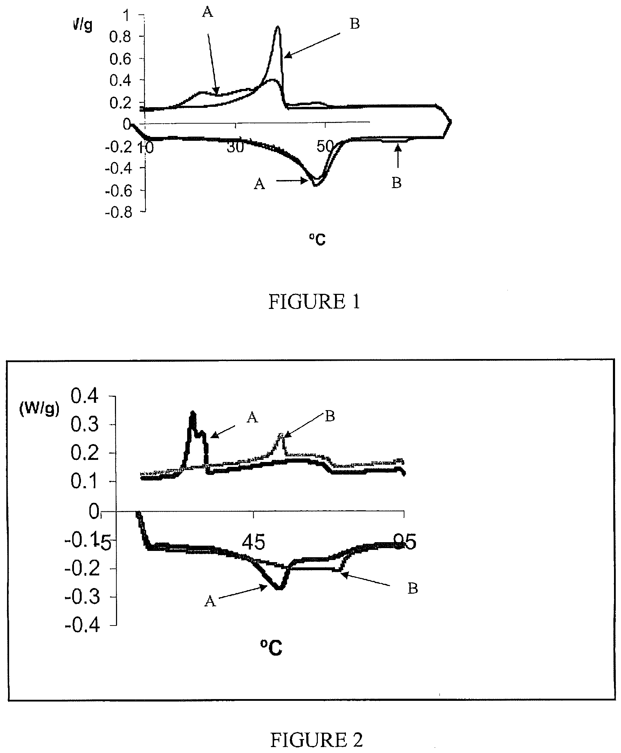

DSC (Differential Scanning Calorimetry) was measured using a TA instrument 2920 MDSC. Both samples showed one crystallization peak having a peak temperature of 39.5°C. For the hydrogenated soybean oil sample, the area under this peak was reduced in half and two additional broad peaks were observed at lower temperatures. Unlike the CASTORWAX™ MP80 only containing sample, no peak corresponding to the crystallization of hydrogenated soybean oil was observed. It is theorized that the hydrogenated soybean oil experiences "super-cooling" and can co-crystallize with stearyl alcohol, which is shown by the additional peaks at lower temperature in Figure 1. The DSC graph is shown in Figure 1 as heat flow (W/g) vs. Temperature (°C). The curves shown correspond to the compositions shown above.

The difference in composition structure can also be seen in the compression values. In Example 3 below, the compression values change as the amount of hydrogenated soybean oil changes.

When using the hydrogenated soybean oil as a co-gellant with the fatty alcohol gellant, the composition has increased fragrance retention as compared to compositions containing hydrogenated castor oil. This effect is shown below in Example 4. After aging, the compositions containing hydrogenated soybean oil had more fragrance remaining in the composition as measured using head space analysis. In one embodiment, the composition has an average fragrance intensity of at least 5x10⁵ µVs as measured using the procedure in Example 4. In another embodiment, the average fragrance intensity is at least about 5.1x10⁵ µVs, at least about 5.2 x10⁵ µVs, at least about 5.5 x10⁵ µVs, or at least about 5.8x10⁵ µVs.

When the hydrogenated soybean oil is used in combination with the hydrocarbon based gellant, the structure, as measured by compression force, is increased as compared to a composition that contains hydrogenated castor oil. When hydrogenated soybean oil replaces hydrogenated castor oil (CASTORWAX™ MP80) in an equal amount by weight in a composition with all other materials remaining the same (see Example 5 below), the ratio of the compression force of the composition with the hydrogenated soybean oil to the compression force of the composition with the hydrogenated castor oil is greater than 1.3. In other embodiments, the ratio is greater than 1.4, 1.5, or 1.6.

In one embodiment, the compression force of the composition is at least about 3500g. In other embodiments, the compression force is at least about 4000g, at least about 4500g, at least about 5000g, at least about 6000g, at least about 7000g, at least about 8000g, at least about 9000g. In another embodiment, the compression force is about 3500g to about 10,000g.

DSC (Differential Scanning Calorimetry) was measured using a TA instrument 2920 MDSC. In the sample using hydrogenated soybean oil, the crystallization is deferred to a lower temperature. The melting and crystallization peaks are stronger with hydrogenated soy, which indicates a more crystalline composition. The DSC graph is shown in Figure 2 as heat flow (W/g) vs. Temperature (°C). The curves shown correspond to the compositions shown below.

| | Sample A | Sample B |
|---|---|---|
| Polyethylene 400 | 10 | 10 |
| H-soybean oil | 6.5 | 0 |
| CASTORWAX™ MP80 | 0 | 6.5 |
| Cyclomethicone | 42.5 | 42.5 |
| C12-15 alkyl benzoate | 15 | 15 |
| Al Zr Tetrachlorohydrex Gly | 22 | 22 |
| PEG-8 distearate | 4 | 4 |

In one embodiment, the composition can provide a payout of about 0.7 to about 0.9g according to the payout test on the Payout, Glide, and Flakeoff Test Machine. In another embodiment, the composition can provide a glide of about 0.8 to about 1.4g according to the glide test on the Payout, Glide, and Flakeoff Test Machine. In anther embodiment, the composition can provide a flakeoff of less that about 25%. In other embodiments, the flake off is less than about 20, about 15, about 10, or about 5%. In other embodiments, the amount of flakeoff is about 1 to about 6%.

As used in this specification, Payout, Glide, and Flakeoff Test Machine refers to the system described in U.S. Application Serial Nos. 11/971,978, filed on 10 January 2008, 61/015,852, filed on 21 December 2007, and 60/976,527 filed on 1 October 2007. The text from Serial No. is reproduced in the Appendix below. See the following sections in the Appendix for the parameters for measuring payout ([Appendix 0039] and [Appendix 0055]), glide ([Appendix 0039] and [Appendix 0060]), and flakeoff ([Appendix 0036]).

### Volatile silicone

Compositions according to the present invention include a volatile silicone. In one embodiment, the volatile silicone is a volatile cyclic polydimethylsiloxane (cyclomethicone), e.g., cyclopentasiloxane. By volatile material it is meant that the material has a measurable vapor pressure at ambient temperature. Preferably, the volatile cyclic polydimethylsiloxane is cyclomethicone. Various types of cyclomethicones may be used. Illustratively, and not by way of limitation, the volatile silicones are one or more members selected from cyclic polydimethylsiloxanes such as those represented by Formula I: 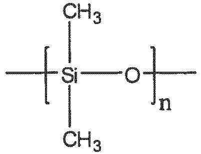 where n is an integer with a value of 3-7, particularly 5-6. Illustrative examples of suitable cyclomethicones are DC-345 and DC-245, manufactured by Dow Corning Corporation, Midland, MI. These types include a tetramer (octylmethylcyclotetrasiloxane) and a pentamer (decamethylcyclopentasiloxane). In one embodiment, the amount of volatile silicone in the composition is 5 to 70% by weight of the composition. In another embodiment, the amount is about 25 to about 45% by weight.

### Antiperspirant active materials

When the composition includes an antiperspirant active, any of the known antiperspirant active materials can be utilized in the composition. Antiperspirant actives include, but are not limited to, aluminum chlorhydrate, aluminum chloride, aluminum sesquichlorohydrate, aluminum-zirconium hydroxychlorides, complexes or adducts of the above-mentioned active ingredients with glycol, such as propylene glycol (for example, "Rehydrol" II from Reheis Chemical Co.), and combinations thereof. Known aluminum-zirconium salts in combination with neutral amino acids, such as glycine (e.g., aluminum-zirconium tetrachlorohydrex Gly) can also be used. Generally, any of the Category I active antiperspirant ingredients, listed in the Food and Drug Administration's Monograph on Antiperspirant Drug Products for overall-the-counter human use (Oct. 10, 1973) can be used.

In other embodiments, the antiperspirant active is an aluminum salt and/or an aluminum-zirconium salt, such as those described above, that are further stabilized by betaine and a calcium salt. More information about betaine and calcium salt stabilized antiperspirant salts can be found in U.S. Patent Application Publication No. 2006/0204463 to Tang et al..

In other embodiments, the antiperspirant active, such as those described above, is selected to have a low metal to chloride ratio. Examples of these antiperspirant actives can be found in U.S. Patent No. 6,375,937 to Chopra et al. and in U.S. Patent Application Publication No. 2004/0109833 to Tang et al..

In other embodiments, the type of salt of interest, an aluminum zirconium tetrasalt or octasalt free of glycine are used wherein aluminum zirconium salt is stabilized by Betaine and has a metal to chloride ratio of about 0.9:1 to about 1.3:1 (and in other embodiments of about 0.9:1 to about 1.2:1 or about 0.9:1 to about 1.1:1). For the tetrasalt, the Al/Zr atomic ratio can be about 3.2:1 to about 4.1:1.0 and the Betaine:zirconium mole ratio can be about 0.2:1 to about 3.0:1 (or in other embodiments of about 0.4:1 to about 1.5:1). Another salt that can be used is an aluminum chloride salt buffered by Betaine, wherein the salt has a metal to chloride ratio of 0.9:1 to 1.3:1 (and in other embodiments of about 0.9:1 to about 1.2:1 or about 0.9:1 to about 1.1:1). For the octasalt the Al/Zr atomic ratio is about 6.2:1 to about 10.0:1 and the Betaine:Zr mole ratio is about 0.2:1 to about 3.0:1 (or in other embodiments of about 0.4:1 to about 1.5:1). In one embodiment, in the case of a salt that contains zirconium, the Betaine is incorporated during the synthesis of the salt so as to maximize the stabilizing effect this ingredient has (especially on the zirconium species). Alternatively, it can be post added to a glycine-free salt along with additional active phase ingredients to form a Betaine stabilized active.

Examples of commercially available glycine-free low M:Cl ratio tetrasalts and octasalts include, but are not limited to, REZAL™ AZP 955 CPG and REZAL™ AZP 885 respectively (both from Reheis Chemical Company, Berkeley Heights, NJ). A more detailed description of making such commercially available salts can be found for example, in U.S. Patent Nos. 7,074,394 and 6,960,338. Further examples of making these types of salt complexes are described in U.S. Patent Application Publication No. 2004/0198998 and United States Patent No. 7,105,691.

In addition to the anti-irritation properties of Betaine, it has also been found that antiperspirant formulations preserve their fragrance stability upon ageing when the Al/Zr salt is used in association with Betaine.

Additionally, the antiperspirant active can be a calcium salt stabilized antiperspirant active. Examples of calcium salt stabilized antiperspirant actives can be found in U.S. Patent Application Publication No. 2006/0204463.

In addition, any new ingredient, not listed in the Monograph, such as aluminum nitratohydrate and its combination with zirconyl hydroxychlorides and nitrates, or aluminum-stannous chlorohydrates, can be incorporated as an antiperspirant active. Antiperspirant actives can include, but are not limited to, the following: astringent salt of aluminum, astringent salt of zirconium, aluminum bromohydrate, aluminum chlorohydrate, aluminum dichlorohydrate, aluminum sesquichlorohydrate, aluminum chlorohydrex PG, aluminum dichlorohydrex PG, aluminum sesquichlorohydrex PG, aluminum chlorohydrex PEG, aluminum dichlorohydrex PEG, aluminum sesquichlorohydrex PEG, aluminum chloride, aluminum sulfate, aluminum zirconium chlorohydrate, aluminum zirconium trichlorohydrate, aluminum zirconium tetrachlorohydrate, aluminum zirconium pentachlorohydrate, aluminum zirconium octachlorohydrate, aluminum zirconium tetrachlorhydrex propylene glycol, aluminum zirconium trichlorohydrex Gly, aluminum zirconium tetrachlorohydrex Gly, aluminum zirconium pentachlorohydrex Gly, aluminum zirconium octachlorohydrex Gly, buffered aluminum sulfate, potassium alum, sodium aluminum chlorohydroxy lactate. In one embodiment, the antiperspirant active is aluminum chlorhydrate. In another embodiment, the antiperspirant active is aluminum zirconium tetrachlorhydrex propylene glycol.

### Deodorant active materials

Any known deodorant active can be used. Examples of deodorant active include, but are not limited to, antimicrobial actives, alcohols, 2,4,4'-trichloro-2'-hydroxy diphenyl ether (Triclosan), benzethonium chloride, polyhexamethylene biguanides, triethylcitrate, 2-amino-2-methyl-1-propanol (AMP), cetyl-trimethylammomium bromide, cetyl pyridinium chloride, farnesol (3,7,11-trimethyl-2,6,10-dodecatrien-1-ol), bactericides, and/or bacteriostats.

### Emollients

The composition can contain emollients in any desired amount to achieve a desired emollient effect. Emollients are known in the art and are used to impart a soothing effect on the skin. Non-volatile emollients are preferable in the present invention. Classes of non-volatile emollients include non-silicone and silicone emollients. Non-volatile, non-silicone emollients include C₁₂₋₁₅ alkyl benzoate. The non-volatile silicone material can be a polyethersiloxane, polyalkyarylsiloxane or polyethersiloxane copolymer. An illustrative non-volatile silicone material in the present invention is phenyl trimethicone. Non-limiting examples of emollients can be found in United States Patent No. 6,007,799. Examples include, but are not limited to, PPG-14 butyl ether, PPG-3 myristyl ether, stearyl alcohol, stearic acid, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl) adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, hydrocyethyl stearate amide.

The composition can additionally include ionizable inorganic salts. These ionizable salts are of the form MₐX_{b} where a=1, or 2 and b=1 or 2; M is a member chosen from Na⁺¹, Li⁺¹, K⁺¹, Mg⁺², Ca⁺², Sr⁺², and Zn⁺² and X is a member chosen chloride, bromide, iodide, citrate, gluconate, lactate, glycinate, glutamate, ascorbate, aspartate, nitrate, phosphate, hydrogenphosphate, dihydrogenphosphate, formate, maloneate, maleate, succinate, carbonate, bicarbonate, sulfate, and hydrogensulfate. In certain embodiments, the selected salts are chosen from NaCl and ZnCl₂. As will be appreciated by those skilled in the art, while it may be possible under certain circumstances to add a salt directly to a portion of the mixture during manufacturing, it is desired to add the salt as a mixture or solution of the salt in a carrier or solvent, particularly water. Of course various concentrations of the salt premix can be made.

The composition may also contain particulates which include but are not limited to talc, mica, fragrance encapsulates, or hydrophobically modified starches, such as aluminum starch octenyl succinate (MACKADERM™ ASTRO-DRY™ from McIntyre Group Ltd.). If the composition is in a liquid form and dispensed through a roll-on applicator, the average particle size of the suspended material is sized so that it can pass through the application to prevent the ball applicator from malfunctioning. Usually, the average particle size does not exceed 150 microns.

In certain embodiments, the composition may also contain as an optional ingredient at least one malodor counteracting alpha, beta-unsaturated ester or mixtures of such materials. In certain embodiments, the level of malodor counteracting composition to deliver a perceivable odor control benefit when delivered from an antiperspirant and/or deodorant composition is about 0.05 to about 0.45 weight % based on the entire composition. The alpha, beta-unsaturated ester malodor counteracting materials are incorporated within the oil phase of an antiperspirant composition. Example of these malodor counteracting components can be found in U.S. Patent No. 6,610, 648 and U.S. Patent No. 6,495,097. For example, in this invention the odor neutralizing alpha, beta unsaturated ester mixture demonstrates unexpected stability in antiperspirant compositions containing low metal:chloride (M:Cl) ratio salts free of glycine. Examples of the alpha, beta unsaturated ester can be found in WO2005/025523, which was filed in the United States as U.S. Application No. 10/571,488.

Examples of the alpha, beta unsaturated ester include, but are not limited to,:
(1) 3-phenyl-2-propenoic acid alkyl esters wherein R¹ is a substituent on the benzene ring and is chosen from an alkyl, an alkoxy, an aryl, or a substituted aryl. In certain embodiments, R¹ is chosen from H, a C₁ to C₈ alkyl, a C₁ to C₈ alkoxy, or an aryl; and R² is a subsistent group replacing the carboxylic acid hydrogen to form the ester where R² has greater than 6 carbon atoms, an aryl, or a substituted aryl group, in certain embodiments R² is a C₆ to C₁₂ alkyl or is a benzyl group; and
(2) an ester of fumaric or maleic acid having linear ester carbon chains from 3-9 carbons, for example dihexyl fumarate;
(3) e-phenyl propenoic acid ester chosen from octyl methoxy cinnamate, phenylethyl cinnamate, benzyl cinnamate;
(4) an aliphatic unsaturated ester, such as dihexyl fumarate.

The composition may optionally further comprise absorbent materials such as corn starch, talc, clay, sodium polyacrylate and/or cotton fiber; and/or other materials such as fragrances, bacteriostats and/or bacteriosides, colorants, etc. Known bacteriostats include baceteriostatic quaternary ammonium compounds such as 2-amino-2-methyl-1-propanol (AMP), cetyl-trimethylammomium bromide, cetyl pyridinium chloride, 2,4,4N-trichloro-2N-hydroxydiphenylether (Triclosan), etc. and various zinc salts.

Antioxidants may be added to the composition, preferably to act as ingredient protectants and for maintenance of long-term stability of the composition. Suitable antioxidants include Tinogard, manufactured by Ciba Specialty Chemicals, Basel, Switzerland.

The compositions as provided herein are described and claimed with reference to their ingredients, as is usual in the art. As would be evident to one skilled in the art, ingredients may in some instances react with one another, so that the true composition of the final formulation may not correspond exactly to the ingredients listed. Thus, it should be understood that the invention extends to the product of the combination of the listed ingredients.

The compositions of the present invention may be manufactured using methods known in the art. Typically, the ingredients are combined and heated to melt the components (other than inert filler), and the melted components (together with particulate inert filler) are mixed. Desirably, volatile materials, such as the fragrance materials, are incorporated in the composition in the latter stages of the mixing cycle, in order to avoid volatilization thereof. After mixing, the molten composition can be poured directly into the dispensers, after which the compositions harden into a solid, and the container is capped to preserve the product until use.

In the following are set forth examples of the present invention. These examples are illustrative, and not limiting, of the present invention. In the following examples, all amounts are in percent of the total weight of the composition.

The hydrogenated castor oil in the examples is CASTORWAX™ from CasChem, Inc.. The product number after the name indicates the melting point of the hydrogenated castor oil. The iodine value of the hydrogenated soybean oil used in the examples is greater than 0 to 1. The weight average molecular weight of the polyethylene used in the examples is indicated by the product number.

To make the compositions, emollients are placed in a 600 ml beaker. The emollients are heated with stirring to 65°C. The gellants are added, and the mixture is heated to 82-85°C. The mixture is cooled to about 80°C, and cyclomethicone, which is preheated to about 70°C, is added. The mixture is cooled to about 75°C and the antiperspirant is added. The temperature is increased to about 80°C and held for about 10 minutes, and the remaining ingredients are added and mixed for one minute. The mixture is poured into oval containers of the type used for antiperspirants/deodorants, and they are placed in a refrigerator at 4°C for 15 minutes. Cooling is completed at room temperature.

Compression is measured using a Texture Analyzer (model #TA-XT21 from Texture Technologies Corp) fitted with a 19 mm square end probe. The antiperspirant stick is removed from the barrel and placed in a hardness sample holder. The sample is positioned so that 2.54 cm (1 inch) of the sample, measured at edge of domed portion is exposed from the Compression Holder for the test. The cover on the hardness holder is closed, and the holder is positioned so that the blade will come in contact at the midpoint of the exposed sample. The instrument is set to run at 1.0 mm/s at a distance of 5.0 mm. The peak value of the compression curve is recorded as the stick hardness value in grams.

### EXAMPLE 1

| Stick Composition | Control | With Hydrogenated Soybean Oil |
|---|---|---|
| Cyclomethicone DC245 | 34.76 | 34.76 |
| C₁₂₋₁₅ alkyl benzoate | 10.19 | 10.19 |
| Stearyl alcohol | 19.22 | 19.22 |
| CASTORWAX™ MP80 | 6.12 | 0 |
| PEG-8 distearate | 3.06 | 3.06 |
| Hydrogenated soybean oil | 0 | 6.12 |
| Antiperspirant Z576 | 20.38 | 20.38 |
| PPG-14 butyl ether | 4.08 | 4.08 |
| Talc | 2.04 | 2.04 |
| Behenyl alcohol | 0.15 | 0.15 |
| Compression (grams force) | 3629 | 3729 |

Example 1 shows that in a stick composition made with replacing CASTORWAX™ with hydrogenated soybean oil in an equal amount resulted in a stick with a comparable compression force (structure).

### EXAMPLE 2

| | Control | With hydrogenated soybean oil |
|---|---|---|
| Phenyl trimethicone | 5 | 5 |
| Cyclomethicone | 36.9 | 36.9 |
| Stearyl alcohol | 18 | 18 |
| Hydrogenated castor oil MP70 | 2 | 0 |
| Hydrogenated castor oil MP90 | 2 | 0 |
| PEG-8 distearate | 2 | 2 |
| PPG-14 butyl ether | 12.1 | 12.1 |
| Antiperspirant AZP908 | 22 | 22 |
| Hydrogenated soybean oil | 0 | 4 |
| Compression (grams force) | 2550 | 1350 |
| Product form | stick | soft solid |

Example 2 shows that it is possible to change the product form by using hydrogenated soybean oil depending on the formulation.

### EXAMPLE 3

The compression force was measured for several compositions containing stearyl alcohol as the gellant with varying amounts of hydrogenated castor oil and hydrogenated soybean oil. The compositions are shown in the table below along with the compression measurements. The results show that the using hydrogenated soybean oil in combination with a fatty alcohol gelling agent, the structure of the composition is changed as indicated by the compression values.

| | 3A | 3B | 3C | 3D |
|---|---|---|---|---|
| PPG-14 butyl ether | 6 | 6 | 6 | 6 |
| C12-15 alkyl benzoate | 10 | 10 | 10 | 10 |
| Hydrogenated soybean oil | 0 | 2 | 5 | 7 |
| CASTORWAX™ MP80 | 7 | 5 | 2 | 0 |
| PEG-8 distearate | 3 | 3 | 3 | 3 |
| Stearyl alcohol | 16 | 16 | 16 | 16 |
| Behenyl alcohol | 0.15 | 0.15 | 0.15 | 0.15 |
| Cyclomethicone | 33.95 | 33.95 | 33.95 | 33.95 |
| Talc | 2 | 2 | 2 | 2 |
| Al Zr Tetrahydrachlorex Gly | 20 | 20 | 20 | 20 |
| Fragrance | 1 | 1 | 1 | 1 |
| Encapsulated Fragrance | 0.9 | 0.9 | 0.9 | 0.9 |
| Compression (g) | 3354.07 | 3262.29 | 2121.8 | 363.5 |
| Standard Deviation | 35.44 | 83.38 | 303.85 | 60.45 |

### EXAMPLE 4

The formula in the table below is used for Example 4. Samples are prepared with no hydrogenated castor oil and no hydrogenated soybean oil, 4% or 8% by weight hydrogenated castor oil (CASTORWAX™ MP80), and 4% or 8% by weight hydrogenated soybean oil.

| | Weight % |
|---|---|
| Cyclomethicone | 35.6 |
| C12-15 alkyl benzoate | 10 |
| PPG14 butyl ether | 5 |
| PEG-8 distearate | 3 |
| Talc | 2 |
| Antiperspirant AZP908 | 20 |
| CASTORWAX™ MP80 | 0, 4, or 8 |
| Hydrogenated soybean oil | 0, 4, or 8 |
| Stearyl alcohol | Balance |

A layer of AP stick under study (0.3 gram) is applied evenly on the surface of a 2.54 cm x 5.08 cm (1"x2") wool flannel (style 527 from Testfabrics Inc., West Pittston, PA). The samples are then placed into a 37°C oven. After 5 hours, the samples are taken out of the oven and sealed into glass vials for Headspace Gas Chromatography analysis (Perkin Elmer HS 40xl headspace sampler coupled with Varian Star 3400 CX Gas Chromatograph). Each formula has 3 replicates. The average for each is reported in the table below.

| Formulas | Averaged fragrance intensity (5 hour aging), x10⁵µVs |
|---|---|
| No co-gellant | 5.8 |
| 4% CASTORWAX™ MP80 | 4.9 |
| 8% CASTORWAX™ MP80 | 4.1 |
| 4% hydrogenated soybean oil | 5.8 |
| 8% hydrogenated soybean oil | 5.2 |

The results above show that formulations using hydrogenated soy as a co-gellant retain more fragrance in the composition after aging as compared to formulations containing castor wax.

### EXAMPLE 5

| | 5A | 5B | 5C | 5D | 5E | 5F |
|---|---|---|---|---|---|---|
| Cyclomethicone DC345 | 42 | 42 | 42 | 42 | 42 | 42 |
| Polyethylene PE400 | 5 | 5 | 12 | 12 | 0 | 0 |
| Polyethylene PE500 | 5 | 5 | 0 | 0 | 12 | 12 |
| Polyethylene PE655 | 2 | 2 | 0 | 0 | 0 | 0 |
| H-soybean oil | 0 | 5 | 0 | 5 | 0 | 5 |
| CASTORWAX™ MP80 | 5 | 0 | 5 | 0 | 5 | 0 |
| C12-15 alkyl benzoate | 15 | 15 | 15 | 15 | 15 | 15 |
| Antiperspirant AZP910 Gold | 22 | 22 | 22 | 22 | 22 | 22 |
| PEG-8 distearate | 4 | 4 | 4 | 4 | 4 | 4 |
| Compression (g force) | 2912 | 4841 | 3205 | 5112 | 3072 | 4550 |

In Example 5, it can be seen that the replacement of CASTORWAX™ (hydrogenated castor oil) with the hydrogenated soybean oil in equal amounts resulted in increased structure as measured by the compression force.

Examples 6-8 below show further compositions according to the invention.

### EXAMPLE 6

| | Wt. % |
|---|---|
| Aluminum-zirconium tetrachlorohydrex gly | 20-21 |
| Stearyl alcohol | 19-20 |
| Cyclomethicone | 34-35 |
| PPG-14 butyl ether | 4-6 |
| PEG-8 distearate | 1-3 |
| C₁₂₋₁₅ Alkyl benzoate | 8-10 |
| Hydrogenated soybean oil | 5-7 |
| Talc | 2-4 |
| Behenyl alcohol | 0.1-0.3 |

### EXAMPLE 7

| | Wt. % |
|---|---|
| Aluminum-zirconium tetrachlorohydrex gly | 20-21 |
| Stearyl alcohol | 15-16 |
| Hydrogenated castor oil | 3-5 |
| Cyclomethicone | 36-38 |
| Myristyl Myristate | 1-3 |
| PEG-8 distearate | 1-3 |
| C₁₂₋₁₅ Alkyl benzoate | 7-9 |
| Hydrogenated soybean oil | 1-3 |
| PPG-3 myristyl ether | 5-7 |
| Talc | 2-4 |
| Behenyl alcohol | 0.1-0.3 |

### EXAMPLE 8

| | wt. % |
|---|---|
| Aluminum-zirconium tetrachlorohydrex gly | 20-21 |
| Stearyl alcohol | 20-21 |
| Hydrogenated castor oil | 4-6 |
| Cyclomethicone | 33-35 |
| PEG-8 distearate | 1-3 |
| Phenyl trimethicone | 1-3 |
| C₁₂₋₁₅ Alkyl benzoate | 4-6 |
| Hydrogenated soybean oil | 7-9 |
| Talc | 2-4 |
| Behenyl alcohol | 0.1-0.3 |

### APPENDIX

**[Appendix 0001]** The description below is the text from U.S. Application No. 11/971,978, filed on 10 January 2008. This text is being included as a description of how to run the payout, glide, and flakoff tests on the described Test Machine. All terms and definitions in this Appendix Section only apply to the Test Machine.

**[Appendix 0002]** In an embodiment of the present invention, a system for measuring any or all of payout, static friction and kinetic friction is disclosed. The system includes at least one substrate positioned on an XYZ translational substrate bed. The system includes a sample holder for supporting a sample, wherein the sample holder and the sample are positioned perpendicular to the XYZ translational substrate bed. The system further includes a force device placing a predetermined weight onto the sample holder; the predetermined weight determines a contact force placed by the sample onto the substrate. The system also includes frictionless bearing table connected to the sample holder and a stationary frictionless bearing table positioned parallel to the XYZ translational substrate bed. The sample holder and the stationary frictionless bearing table are connected to a friction sensor. The system also includes a balance for obtaining a first substrate weight before movement of the XYZ translational substrate bed and a second substrate weight after movement of the XYZ translational substrate bed.

**[Appendix 0003]** The system further includes a controller operably coupled to the moving substrate bed and the friction sensor and configured to execute a machine readable program code containing executable instructions.

**[Appendix 0004]** In an embodiment of the present invention, a method for measuring payout is disclosed. The method comprises positioning a substrate of pre-known weight on an XYZ translational substrate bed; supporting a sample in a sample holder, wherein the sample is perpendicular to the XYZ translational substrate bed; placing a predetermined weight onto the sample holder so that the sample and substrate form a contact point; first moving the XYZ translational substrate bed at a first sweep speed in a first direction relative to the sample; second moving the XYZ translational substrate bed at a second sweep speed in a second direction relative to the sample; conducting the first moving and the second moving for a predetermined number of cycle(s); obtaining a second substrate weight of the substrate after the predetermined number of cycles; and determining a payout value based on the first substrate weight and the second substrate weight.

**[Appendix 0005]** In an embodiment of the present invention, a method for measuring one or more of static friction and kinetic friction is provided. The method comprises: positioning a substrate of pre-known weight on an XYZ translational substrate bed; supporting a sample in a sample holder, wherein the sample is perpendicular to the XYZ translational substrate bed; placing a predetermined weight onto the sample holder so that the sample and substrate form a contact point; first moving the XYZ translational substrate bed at a first sweep speed in a first direction relative to the sample; second moving the XYZ translational substrate bed at a second sweep speed in a second direction relative to the sample; conducting the first moving and the second moving for a predetermined number of cycle(s); during the first moving step and the second moving step, measuring one or more friction values at the contact point; analyzing one or more friction values generated at the sample contact point during the first moving step and the second moving step; and determining one or more of a static friction value and a kinetic friction value based on the one or more friction values.

**[Appendix 0006]** In an embodiment of the present invention, a method for measuring flakeoff is provided. The method comprises: providing a wool sample of a predetermined size; applying an initial weight of a material to the wool sample; attaching a first end of the wool to a stationary holder and a second end to a movable substrate bed; a stretching step comprising moving the movable substrate bed a predetermined distance and returning and then moving it to an opposite direction for the same predetermined distance and returning for 1 stretch; repeating the stretch step for a predetermined number of stretches; measuring the weight of the wool sample and material after the predetermined number of stretches; determining a weight loss of material from the wool sample as measured by an amount of material lost from the sample divided by the initial weight of material after the predetermined number of stretches.

**[Appendix 0007]** In each of the above methods, the methods are conducted on the above described system.

### BRIEF DESCRIPTION OF DRAWINGS

**[Appendix 0008]** Reference will now be made in detail to embodiments of the present disclosure, examples of which are illustrated in the accompanying drawings. Wherever possible, the same reference numbers will be used throughout the drawings to refer to the same or like parts.

**[Appendix 0009]** Appendix Figure 1 illustrates an exemplary system to measure payout, static friction, kinetic friction, and combinations thereof.

**[Appendix 0010]** Appendix Figure 2 illustrates an exemplary device to measure payout, static friction, kinetic friction, and combinations thereof.

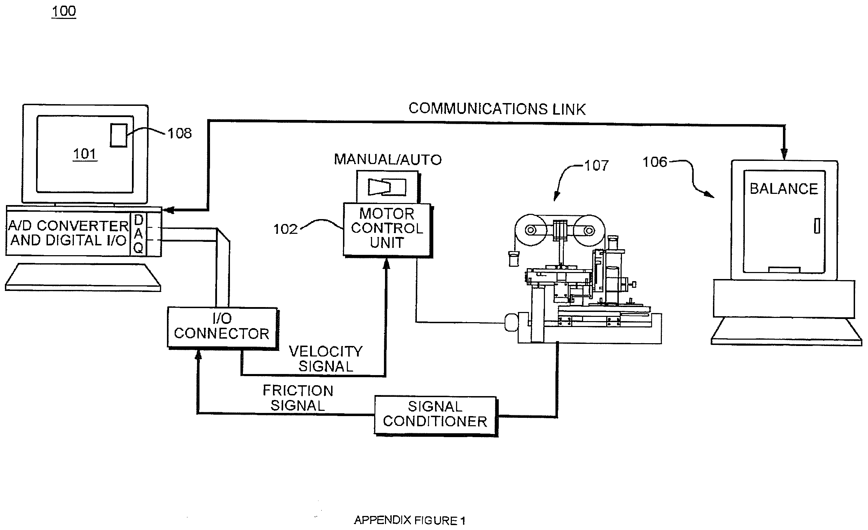

**[Appendix 0011]** Appendix Figure 3 illustrates an exemplary friction sensor.

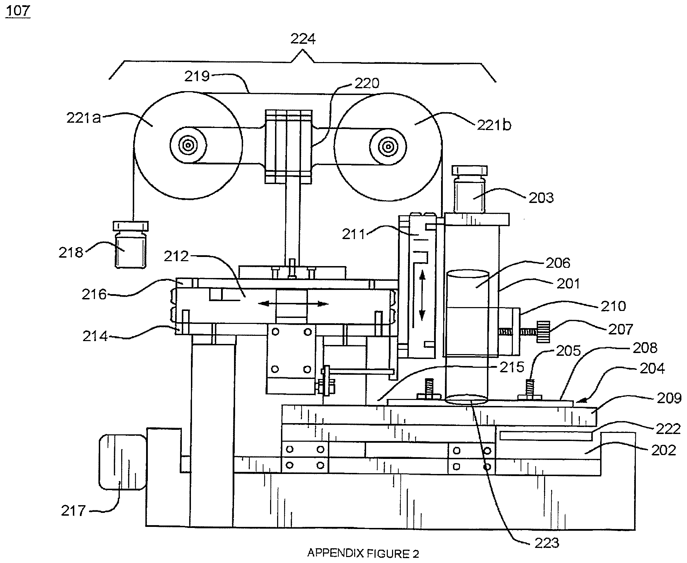

**[Appendix 0012]** Appendix Figure 4 illustrates a model for determining the friction coefficient.

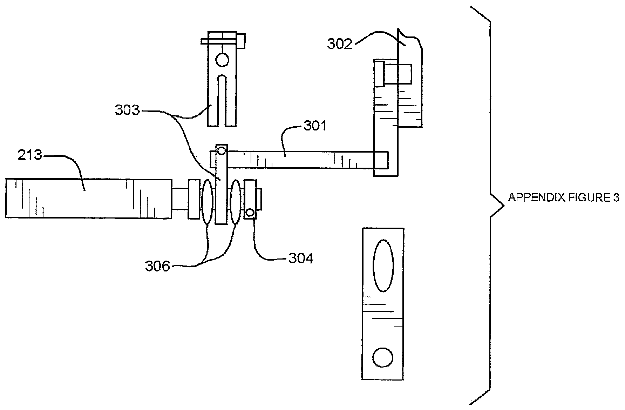

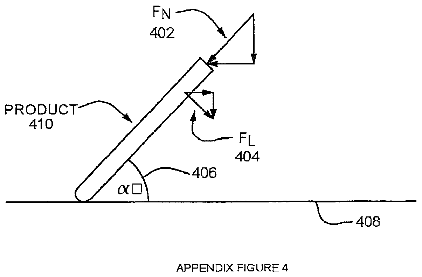

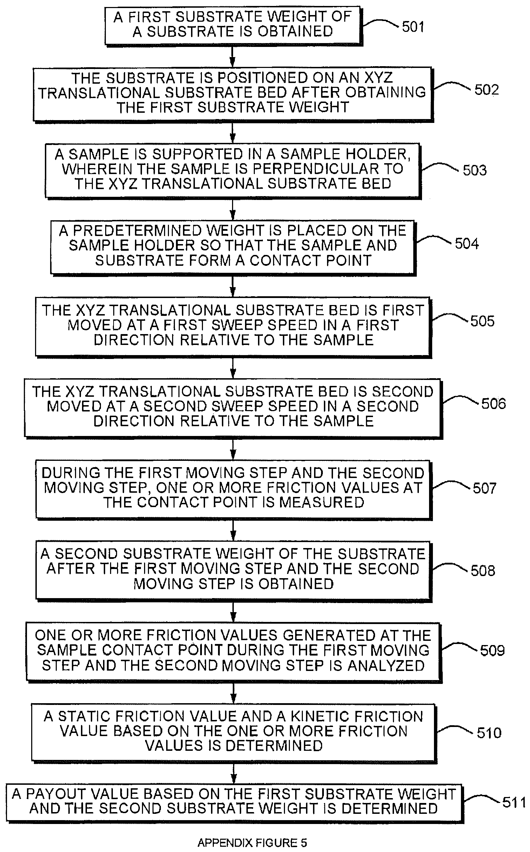

**[Appendix 0013]** Appendix Figure 5 illustrates an exemplary method using the systems described herein.

### DETAILED DESCRIPTION OF THE INVENTION

**[Appendix 0014]** As used throughout, ranges are used as a shorthand for describing each and every value that is within the range. Any value within the range can be selected as the terminus of the range. In addition, all references cited herein are hereby incorporated by reference in their entireties. In the event of a conflict in a definition in the present disclosure and that of a reference, the present disclosure controls.

**[Appendix 0015]** The present invention provides for systems and methods for measuring payout, static friction, kinetic friction or combinations thereof. Appendix Figure 1 illustrates an exemplary system 100 including a payout friction tester device 107, a balance 106, and a controller 101 having a machine readable program code 108 containing executable instructions. The device 107 for measuring payout, static friction, kinetic friction or combinations thereof can be operably linked to the controller 101 through a motor control unit 102. The components of the exemplary system 100 illustrated in Appendix Figure 1 are described further below.

[Appendix 0016] Appendix Figure 2 illustrates an exemplary payout friction device 107. Device 107, of system 100, includes: at least one substrate 204 positioned on an XYZ translational substrate bed 209; a sample holder 201; a force device 224; a frictionless bearing table 211; a stationary frictionless bearing table 212; and a friction sensor 213. Sample holder 201 supports sample 206 so that the sample 206 can be positioned perpendicular to the XYZ translational substrate bed 209 or so that the sample 206 contacts the substrate 204 perpendicularly. The sample holder 201 can also support the sample 206 such that the sample 206 contacts the substrate 204 at an angle that is less than 90°.

[Appendix 0017] Sample 206 can be any sample that can be analyzed for payout, static friction, kinetic friction or combinations thereof. Examples of samples include but are not limited to deodorants (e.g. a deodorant stick), antiperspirants, or combinations thereof. The sample 206 can be secured to the sample holder 201 using a screw 207, such as a knurled thumbscrew, or other means for attachments, such as a clip or other means that can secure the sample 206 and assist in orienting its alignment. The sample clamp 210 can accept deodorant stick canisters 206 or other types of sample containers of various sizes and configurations.

[Appendix 0018] Substrate 204 may include materials such as copier grade paper, sandpaper (in differing grades of abrasion) or cloth may be used. In some embodiments, it is convenient to cut the substrate beforehand in bulk, for example, into approximately 13 x 25 centimeter strips so that single strips can be clamped in place before testing.

[Appendix 0019] Referring again to Appendix Figure 2, the XYZ translational substrate bed 209 functions to move the XYZ translational substrate bed at a first sweep speed in a first direction and at a second sweep speed in a second direction relative to the sample 206. The XYZ translational substrate bed 209 is operably coupled to a motorized screw table 202. The motorized screw table 202 can be driven by an electronic drive unit 217. The electronic drive unit 217 can operate in an automated mode or a manual mode. In the automatic mode, the electronic drive unit 217 can include a pulse width modulation speed control so to achieve precise speed control down to a zero velocity high torque condition. The motor 103 can be remotely driven by a velocity signal furnished by the controller 101, for example by the controller's analog output channel. This allows precise control over the sweep rate and distance. In the manual mode, an operator manipulates the XYZ translational substrate bed 209 using controls of the electronic drive unit 102. An example of an electronic drive unit 217 is, but not limited to, a Motamatic Drive Unit.

**[Appendix 0020]** In one embodiment, the XYZ translational substrate bed 209 also includes a heater 222. In some embodiments, the heater 222 is capable of heating the substrate 204 to a temperature of about 26.7°C to about 43.3°C (about 80°F to about 110°F), about 32.2°C to about 43.3°C (about 90°F to about 110°F), about 32.2°C to about 37.8°C (about 90°F to about 100°F), about 35°C to about 37.8°C (about 95°F to about 100°F), about 36.7°C to about 37.8°C (about 98°F to about 100°F), 36.7°C to about 37.2°C (about 98°F to about 99°F), or about 37°C (about 98.6°F).

[Appendix 0021] Frictionless bearing table 211 is connected to the sample holder 201 permitting "frictionless" movement of the sample 206 supported by the sample holder 201. In some embodiments, the frictionless bearing table 211 is positioned perpendicular to the XYZ translational substrate bed 209. In other embodiments, the frictionless bearing table 211 is positioned vertically. The frictionless bearing table 211 functions to maintain an axis of pressure with testing and permits up and down movement of the sample holder 201. The weight of the sample holder 201 can be counter balanced to zero force through counterweight 218 via the pulley tower 220 and cable 219. Additional weight(s) 203 are placed on top of the sample holder 201 to define the magnitude of contact force (that which presses the sample against the surface).

[Appendix **0022]** A stationary frictionless bearing table 212 is positioned parallel to the XYZ translational substrate bed 209. In some embodiments, the stationary frictionless bearing table 212 is a horizontal frictionless bearing table. In other embodiments, the stationary frictionless bearing table 212 is positioned on internal rails supported by a plurality of ball bearings. The stationary frictionless bearing table floor 214 is part of the base 216 for device 107 and does not move permitting the measurement of force with respect to a solid reference.

[Appendix **0023]** Friction sensor 213 is operably connected to the sample holder 201 and the stationary frictionless bearing table 209. In one embodiment, friction sensor 213 can be mounted above the XYZ translational bed 209 on a bracket secured to the stationary frictionless bearing table floor 214. Lateral friction is transmitted to the friction sensor 213 through a linkage 215 coupling arrangement. This linkage 215 can be oriented as close as practical to the plane of actual friction. Measuring friction at the sample contact point 223 requires that other friction points in the machine be eliminated or at least minimized as much as possible. To accomplish this, the stationary frictionless bearing table 212 supports the upper assembly completely. All of the assembly components can be bound together on a supporting structure 216 (shown as a sideways T in black). This "rides" as one piece on the stationary frictionless bearing table 212.

**[Appendix 0024]** The friction sensor 213 can be any sensor that can be used to detect and determine friction. Transferring surface friction to the sensing element can be done by a mechanical linkage from the sample holder 201 to the friction sensor 213. Referring to Appendix Figure 3, the friction sensor 213 is operably coupled to a linkage 215 including a transmitter bar 301 and a linkage fork 303. Transmitter bar 301 connects registered force at the sample contact point 223 (Appendix Figure 2) from the sample carriage mount 302 to the linkage fork 303. The linkage fork 303 can be positioned between a pair of O-ring dampeners 306 and the pair of O-ring dampeners can be positioned between a pair of element stops 304. The linkage fork 303 is suspended between two element stops 304 attached to the friction sensor probe 305. When the linkage fork 303 pushes against a stop its force content is transferred to the friction sensor 213. Physical contact at the stops is intentionally dampened by rubber "O" rings 306 which assist in smoothing out the elastic ringing that results from abrupt changes in force direction

**[Appendix 0025]** Referring again to Appendix Figure 2, device 107 can include a force device 224 including a predetermined weight 203, a counter weight 218, a cord 219, a pulley tower 220, and two pulleys 221a and 221b. Force device 224 functions to place a predetermined weight 203 onto sample holder 201 where the predetermined weight 203 determines a contact force placed by the sample 206 onto the substrate 204. The predetermined weight 203 and the counter weight 218 can be connected by the cord 219. In some embodiments, the stationary frictionless bearing table 212 supports force device 224.

**[Appendix 0026]** Referring to both Appendix Figure 1 and Appendix Figure 2, system 100 may also include a controller 101. for monitoring and controlling the desired variables. Any type of controller can be used to operate the system. Installed in the controller is a multi-functional A/D converter card (DAQ) providing the necessary interface to the system to the various components. Controller 101 is operably coupled to the XYZ translational substrate bed 209, the balance 106, and the friction sensor 217 and configured to execute the machine readable program code 108. Controller 101 is configured to execute machine readable program code 108 to perform various functions. In some embodiments, the functions include, but are not limited to configuring the balance 106 to obtain the first substrate weight before movement of the XYZ translational substrate bed 209 and the second substrate weight after movement of the XYZ translational substrate bed 209. Controller 101 also configures the XYZ translational substrate bed 209 to move the XYZ translational substrate bed 209 at a first sweep speed in a first direction and at a second sweep speed in a second direction relative to the sample 206. Controller 101 also analyzes one or more friction values, measured by the friction sensor, generated at the sample contact point 223 located between the sample 206 and the substrate 204 during movement of the XYZ translational substrate bed 209. Controller 101 is further configured to perform the determine a static friction value and a kinetic friction value based on the one or more friction values or determine a payout value based on the first substrate weight and the second substrate weight.

**[Appendix 0027]** The system of the present invention can also be configured to execute machine readable code containing executable program instructions to perform a variety of functions. In some embodiments, the system is configured to perform methods for measuring one or more of the following: payout, static friction and kinetic friction. One embodiment for measuring one or more of the following: payout, static friction and kinetic friction is illustrated in Appendix Figure 5. In step 501, a first substrate weight of a substrate is obtained. In one embodiment, a fresh piece of substrate 204 is placed into the balance 106 to be weighed. A continuous reading from the balance 106 is displayed in the window as the balance 106 is loaded. Once a stable reading is noted it can be "acquired" by pushing an on screen button labeled "Get weight". The substrate 204 is then removed from the balance 106 and secured to the XYZ translational bed 209 with clamping plates 208 on the longitudinal sides.

**[Appendix 0028]** In step 502 the substrate is positioned on an XYZ translational substrate bed after obtaining the first substrate weight. In step 503 a sample is supported in a sample holder, wherein the sample is perpendicular to the XYZ translational substrate bed. In Step 504 a predetermined weight is placed onto the sample holder so that the sample and substrate form a contact point.

**[Appendix 0029]** In step 505 the XYZ translational substrate bed 209 is first moved at a first sweep speed in a first direction relative to the sample. In step 506 the XYZ translational substrate bed is second moved at a second sweep speed in a second direction relative to the sample. In one embodiment, controller 101 begins the sweeping process when permission is given by an operator. In another embodiment, controller 100 begins the sweeping process based on an automated process where permission is not needed but instead the process begins when the sample 206 and the substrate 204 are secured. The sweeping steps 505 and 506, are performed by a motorized screw table that is driven by an electronic drive unit. The electronic drive unit can have a pulse width modulation speed control. In some embodiments, the first moving step and the second moving step are repeated a predetermined number of times. In some embodiments, the first moving step and the second moving step are performed 1-50, 1-40, 1-30, 1-20, 1-10, 5-10, 5-15, 5, or 10 times.

**[Appendix 0030]** The distance moved in the first direction or the second direction by the XYZ translational substrate bed 209, during the sweep steps 505 and 506 can be varied. In some embodiments, the distance of the first direction or the second direction is about 5 to about 50 cm, about 5 to about 40 cm, about 5 to about 30 cm, about 5 to about 20 cm, about 5 to about 10 cm. In some embodiments, distance of the first direction or the second direction is about 5, about 10, about 15, about 20, about 25, about 30, about 35, about 40, or about 50 cm.

**[Appendix 0031]** In step 507 during the first moving step and the second moving step, one or more friction values at the contact point is measured. In some embodiments, lateral friction can be measured directly as the XYZ translational substrate bed 209 sweeps in the first and second directions. In one embodiment, each response from the friction sensor 213 can be displayed in real time at controller 101, as the sweeping continues.

**[Appendix 0032]** In step 508 a second substrate weight of the substrate after the first moving step and the second moving step is obtained. When the requested number of sweep steps has occurred the computer can re-display the "Get weight" window. The impregnated material, i.e. substrate 204, can be removed from the lower bed and placed back into the balance 106 to be post-weighed. Payout is determined from the change in weight of the substrate 204.

**[Appendix 0033]** In step 509 one or more friction values generated at the sample contact point during the first moving step and the second moving step is analyzed. In step 510 a static friction value and a kinetic friction value based on the one or more friction values are determined. In some embodiments the friction values are determined using the formula described herein. In step 511 a payout value based on the first substrate weight and the second substrate weight is determined.

**[Appendix 0034]** The present invention also provides for determining friction coefficients as the substrate and sample pass against one another. Using the systems described herein the sample moves or glides across the substrate in a pattern that involves acceleration and de-acceleration unlike the previous assumption that the motion occurs with uniform speed. Therefore, the following model based on Newton's second law was employed to calculate the coefficient of friction between the sample and the substrate. Appendix Figure 4 illustrates a model configuration of the substrate and sample passing against one another where F_{N} is the normal force applied to the skin 408, F_{L} is the net lateral force across the skin 408, α is the angle between the product 410 and the skin 408 at any given time. Based on the configuration displayed in Appendix Figure 4, the friction coefficient at any given time can be express as following:
**[Appendix 0035]** Driving force = F_{L} sin (α) - F_{N} cos (α); Friction Force = µ * [F_{L} cos (α) + F_{N} sin (α)]; Newton's second law: F_{L} sin (α) - F_{N} cos (α) - µ * [F_{L} cos (α) + F_{N} sin (α)] = m*a;
µ = { F_{L} sin (α) - F_{N} cos (α) - m*a}/[F_{L} cos (α) + F_{N} sin (α)]; where m*a is the inertia of the (carriage + sample) times acceleration (a).

**[Appendix 0036]** The device 107 can also be used to measure flakeoff. Flakeoff is a measure of weight loss of material from a sample that has been stretched. It is a measure of how well a material (such as an antiperspirant/deodorant composition) will remain on a substrate. In one embodiment, a predetermined amount of material (for example, 0.65±0.03g) to be tested is applied onto a piece of wool (Style #530 from Testfabrics, Inc.) of a predetermined size (for example, 7.6 cm x 15.2 cm (3 in. x 6 in.)). The wool is stretched a predetermined distance (for example 6 cm) and returned and then stretched to the opposite direction for the same predetermined distance and returned as one stretch. The weight of the wool and material is measured after a predetermined number of stretches (for example 50, 150, and/or 450 stretches). The percent weight loss of the material from the wool is recorded as a measure of flake-off. In one embodiment, the results from four samples can be averaged to give an averaged result. In device 107, one end of the wool is attached to a stationary holder, which is attached to the frictionless bearing table 211 as replacement of sample holder 201, and the other end of the wool is attached to substrate bed 209; oriented across the 15.2 cm length. The wool is thus perpendicular to the substrate bed 209. Substrate bed 209 is then moved to stretch the wool.

### [Appendix 0037] Examples

### [Appendix 0038] Example 1: Payout/Glide on Sample

**[Appendix 0039]** Payout on a sample is measured using the system described herein. The system holds the deodorant stick flush to the substrate and moves the stick with a set speed over a distance of 100 mm with 500g of force. The payout program measures the amount of the product applied to a cotton substrate after 10 strokes, whereas the glide program measures the friction to move the stick across the substrate during one stroke. Immediately prior to payout analysis, three sticks of each experimental stick are cut flat and then the stick surface was is further flattened or conditioned on the instrument using a speed of 30 mm/sec for 20 cycles. In order to determine the payout, the cotton substrate is tared on a balanced and then clamped down on the substrate bed. The stick is passed over the substrate 10 times at a speed of 20 mm/sec, and then the substrate is removed and returned to the balance to obtain the weight of the product on the substrate. The payout is measured three times on a stick and the average of the three results is calculated. The friction coefficient for the first and tenth strokes is recorded.

**[Appendix 0040]** A system for measuring one or more of the following: payout, static friction and kinetic friction comprising:
at least one substrate positioned on an XYZ translational substrate bed;
a sample holder for supporting a sample, wherein the sample holder and the sample are positioned perpendicular to the XYZ translational substrate bed;
a force device placing a predetermined weight onto the sample holder, the predetermined weight determining a contact force placed by the sample onto the substrate;
a frictionless bearing table connected to the sample holder;
a stationary frictionless bearing table positioned parallel to the XYZ translational substrate bed;
a friction sensor connected to the sample holder and the stationary frictionless bearing table;
a balance for obtaining a first substrate weight before movement of the XYZ translational substrate bed and a second substrate weight after movement of the XYZ translational substrate bed;
a machine readable program code containing executable instructions; and
a controller operably coupled to the moving substrate bed, the balance, and the friction sensor and configured to execute the machine readable program code so to perform the following:
   configure the balance to obtain the first substrate weight and the second substrate weight;
   configure the XYZ translational substrate bed to move the XYZ translational substrate bed at a first sweep speed in a first direction and at a second sweep speed in a second direction relative to the sample; and
   analyze one or more friction values, measured by the friction sensor, generated at the sample contact point located between the sample and the substrate during movement of the XYZ translational substrate bed.

**[Appendix 0041]** The system of [Appendix 0040], wherein the sample comprises an antiperspirant or deodorant stick.

**[Appendix 0042]** The system of [Appendix 0040], wherein the XYZ translational substrate bed is operably coupled to a motorized screw table.

**[Appendix 0043]** The system of [Appendix 0042], wherein the motorized screw table is driven by an electronic drive unit.

**[Appendix 0044]** The system of [Appendix 0043], wherein the electronic drive unit has pulse width modulation speed control.

**[Appendix 0045]** The system of [Appendix 0040], wherein the friction sensor is operably coupled to a linkage comprising a transmitter bar and a linkage fork.

**[Appendix 0046]** The system of [Appendix 0045], wherein the transmitter bar is connected to the sample holder and the linkage fork, and wherein the linkage fork is further coupled to the friction sensor.

**[Appendix 0047]** The system of [Appendix 0046], wherein the linkage fork is positioned between a pair of O-ring dampeners and the pair of O-ring dampeners are positioned between a pair of element stops.

**[Appendix 0048]** The system of [Appendix 0040], wherein the force device comprises the predetermined weight, a counter weight, a cord, a pulley tower and two pulleys, wherein the predetermined weight and the counter weight are connected by the cord.

**[Appendix 0049]** The system of [Appendix 0040], wherein stationary frictionless bearing table is positioned on internal rails supported by a plurality of ball bearings.

**[Appendix 0050]** The system of [Appendix 0040], wherein the controller is further configured to perform the following:
based on the one or more friction values, determine a static friction value and a kinetic friction value; and
based on the first substrate weight and the second substrate weight, determine a payout value.

**[Appendix 0051]** The system of [Appendix 0040], wherein the sample holder supports the sample in a vertical position.

**[Appendix 0052]** The system of [Appendix 0040], wherein the force device comprises a vertical force device.

**[Appendix 0053]** The system of [Appendix 0040], wherein the stationary frictionless bearing table comprises a horizontal frictionless bearing table.

**[Appendix 0054]** The system of [Appendix 0040], wherein the XYZ translational substrate bed includes a heater.

**[Appendix 0055]** A method for measuring payout comprising:
positioning a substrate of pre-known weight on an XYZ translational substrate bed;
supporting a sample in a sample holder, wherein the sample is perpendicular to the XYZ translational substrate bed;
placing a predetermined weight onto the sample holder so that the sample and substrate form a contact point;
first moving the XYZ translational substrate bed at a first sweep speed in a first direction relative to the sample;
second moving the XYZ translational substrate bed at a second sweep speed in a second direction relative to the sample;
conducting the first moving and the second moving for a predetermined number of cycle(s);
obtaining a second substrate weight of the substrate after the predetermined number of cycles; and
determining a payout value based on the first substrate weight and the second substrate weight.

**[Appendix 0056]** The method of [Appendix 0055], wherein the first sweep speed and the second sweep speed is 20 mm/s, the predetermined number of cycles is 10, a total distance traveled in one cycle is 100 mm, and the sample is applied to the substrate at a 500 g force.

**[Appendix 0057]** The method of [Appendix 0055] further comprising preconditioning the sample comprising conducting the method in [Appendix 0055] up to the conducting step for 20 cycles at a first sweep speed and second sweep speed of 30 mm/s on a substrate that is then discarded.

**[Appendix 0058]** The method of [Appendix 0055], wherein the XYZ translational substrate bed is heated to a temperature of 37°C.

**[Appendix 0059]** The method of [Appendix 0055], wherein the sample comprises an antiperspirant or deodorant stick.

**[Appendix 0060]** A method for measuring one or more of static friction and kinetic friction comprising:
positioning a substrate of pre-known weight on an XYZ translational substrate bed;
supporting a sample in a sample holder, wherein the sample is perpendicular to the XYZ translational substrate bed;
placing a predetermined weight onto the sample holder so that the sample and substrate form a contact point;
first moving the XYZ translational substrate bed at a first sweep speed in a first direction relative to the sample;
second moving the XYZ translational substrate bed at a second sweep speed in a second direction relative to the sample;
conducting the first moving and the second moving for a predetermined number of cycle(s);
during the first moving step and the second moving step, measuring one or more friction values at the contact point;
analyzing one or more friction values generated at the sample contact point during the first moving step and the second moving step; and
determining one or more of a static friction value and a kinetic friction value based on the one or more friction values.

**[Appendix 0061]** The method of [Appendix 0060] further comprising preconditioning the sample comprising conducting the method in [Appendix 0060] up to the conducting step for 20 cycles at a first sweep speed and second sweep speed of 30 mm/s on a substrate that is then discarded.

**[Appendix 0062]** The method of [Appendix 0060], wherein the XYZ translational substrate bed is heated to a temperature of 37°C.

**[Appendix 0063]** The method of [Appendix 0060], wherein the sample comprises an antiperspirant or deodorant stick.

**[Appendix 0064]** A method of measuring flakeoff of a material comprising:
providing a wool sample of a predetermined size;
applying an initial weight of a material to the wool sample;
attaching a first end of the wool to a stationary holder and a second end to a movable substrate bed;
a stretching step comprising moving the movable substrate bed a predetermined distance and returning and then moving it to an opposite direction for the same predetermined distance and returning for 1 stretch;
repeating the stretch step for a predetermined number of stretches;
measuring the weight of the wool sample and material after the predetermined number of stretches;
determining a weight loss of material from the wool sample as measured by an amount of material lost from the sample divided by the initial weight of material after the predetermined number of stretches.

**[Appendix 0065]** The method of [Appendix 0064], wherein the wool sample measures 7.6 cm x 15.2 cm and the initial weight of material is 0.65±0.03 g.

**[Appendix 0066]** The method of [Appendix 0064], wherein the predetermined distance is 6 cm.

**[Appendix 0067]** The method of [Appendix 0064], wherein the predetermined number of stretches is 50 and optionally one or more of 150 and 450.

## Claims

1. A composition comprising:
i) at least one active chosen from at least one antiperspirant active and at least one deodorant active;
ii) a first gellant comprising at least one fatty alcohol;
iii) at least one soybean oil having an iodine value of 1 to 5 present in an amount of 3 to 7% by weight of the composition, wherein the iodine value is measured by ASTM D5554-95 (2006); and
iv) at least one volatile silicone.

2. The composition of claim 1, wherein the first gellant is present in the composition in an amount of 5 to 25 % by weight of the composition, optionally 10 to 20% by weight.

3. The composition of claim 1, wherein the fatty alcohol comprises stearyl alcohol.

4. The composition of claim 1, wherein the volatile silicone is present in an amount of 5 to 70 % by weight of the composition.

5. The composition of claim 1, wherein the volatile silicone comprises cyclomethicone.

6. The composition of claim 1, wherein the antiperspirant active is present in an amount of 10 to 25 % by weight of the composition.

7. The composition of claim 1, wherein the deodorant active is present in an amount of greater than 0 to 1 % by weight of the composition.

8. The composition of claim 1 further comprising an emollient, optionally wherein the emollient is chosen from PPG-14 butyl ether, C12-15 alkyl benzoate, phenyl trimethicone, PPG-3 myristyl ether, myristyl myristate, and combinations thereof.

9. The composition of claim 1, wherein the composition is a solid stick or soft solid.

10. The composition of claim 1, wherein the composition has a payout of 0.7 to 0.9g according to a payout test on a Payout, Glide, and Flakeoff Test machine.

11. The composition of claim 1, wherein the composition has a glide of 0.8 to 1.4g according to a glide test on a Payout, Glide, and Flakeoff Test machine.

12. The composition of claim 1, wherein the composition has a flakeoff of less than 25%.

## Patentansprüche

1. Zusammensetzung umfassend:
(i) mindestens einen Wirkstoff, ausgewählt aus mindestens einem Antiperspirant-Wirkstoff und mindestens einem Deodorant-Wirkstoff;
(ii) ein erstes Gelliermittel, umfassend mindestens ein Fettalkohol;
(iii) mindestens ein Sojabohnenöl mit einem Jodwert von 1 bis 5, das in einer Menge von 3 bis 7 Gew.% der Zusammensetzung vorliegt, wobei der Jodwert durch ASTM D5554-95 (2006) gemessen ist; und
(iv) mindestens ein volatiles Silikon.

2. Zusammensetzung nach Anspruch 1, wobei das erste Gelliermittel in der Zusammensetzung in einer Menge von 5 bis 25 Gew.% der Zusammensetzung, wahlweise 10 bis 20 Gew.%, vorliegt.

3. Zusammensetzung nach Anspruch 1, wobei der Fettalkohol Stearylalkohol umfasst.

4. Zusammensetzung nach Anspruch 1, wobei das volatile Silikon in einer Menge von 5 bis 70 Gew.% der Zusammensetzung vorliegt.

5. Zusammensetzung nach Anspruch 1, wobei das volatile Silkon Cyclomethicon umfasst.

6. Zusammensetzung nach Anspruch 1, wobei der Antiperspirant-Wirkstoff in einer Menge von 10 bis 25 Gew.% der Zusammensetzung vorliegt.

7. Zusammensetzung nach Anspruch 1, wobei der Deodorant-Wirkstoff in einer Menge von mehr als 0 bis 1 Gew.% der Zusammensetzung vorliegt.

8. Zusammensetzung nach Anspruch 1, des Weiteren umfassend einen Weichmacher, wobei der Weichmacher wahlweise ausgewählt ist aus PPG-14 Butylether, C12-15 Alkylbenzoat, Phenyltrimethicon, PPG-3 Myristylether, Myristylmyristat, und Kombinationen davon.

9. Zusammensetzung nach Anspruch 1, wobei die Zusammensetzung ein fester Stab oder ein weicher Feststoff ist.

10. Zusammensetzung nach Anspruch 1, wobei die Zusammensetzung einen Payout von 0,7 bis 0,9 g in einem Payout-Test mit einer Payout-, Glide-, und Flakeoff-Testmaschine aufweist.

11. Zusammensetzung nach Anspruch 1, wobei die Zusammensetzung einen Glide von 0,8 bis 1,4 g in einem Glide-Test mit einer Payout-, Glide-, und Flakeoff-Testmaschine aufweist.

12. Zusammensetzung nach Anspruch 1, wobei die Zusammensetzung eine Exfoliierung von weniger als 25% aufweist.

## Revendications

1. Composition comprenant :
i) au moins un actif choisi parmi au moins un actif anti-transpirant et au moins un actif déodorant ;
ii) un premier gélifiant comprenant au moins un alcool gras ;
iii) au moins une huile de soja ayant un indice d'iode de 1 à 5, présente en une quantité de 3 % à 7 % en poids de la composition, dans laquelle l'indice d'iode est mesuré par la norme ASTM D5554-95 (2006) ; et
iv) au moins une silicone volatile.

2. Composition selon la revendication 1, dans laquelle le premier gélifiant est présent dans la composition en une quantité de 5 % à 25 % en poids de la composition, éventuellement de 10 % à 20 % en poids.

3. Composition selon la revendication 1, dans laquelle l'alcool gras comprend de l'alcool stéarylique.

4. Composition selon la revendication 1, dans laquelle la silicone volatile est présente en une quantité de 5 % à 70 % en poids de la composition.

5. Composition selon la revendication 1, dans laquelle la silicone volatile comprend de la cyclométhicone.

6. Composition selon la revendication 1, dans laquelle l'actif anti-transpirant est présent en une quantité de 10 % à 25 % en poids de la composition.

7. Composition selon la revendication 1, dans laquelle l'actif déodorant est présent en une quantité supérieure à 0 % jusqu'à 1 % en poids de la composition.

8. Composition selon la revendication 1, comprenant en outre un émollient, éventuellement dans laquelle l'émollient est choisi parmi le PPG-14 butyl éther, un benzoate d'alkyle en C12-15, laphényl triméthicone, le PPG-3 myristyl éther, le myris-tatede myristyle, et des combinaisons de ces derniers.

9. Composition selon la revendication 1, dans laquelle la composition est un bâton solide ou un solide souple.

10. Composition selon la revendication 1, dans laquelle la composition a une distribution de 0,7 à 0,9 g selon un test de distribution sur une machine de test de distribution, de glissement et d'écaillage.

11. Composition selon la revendication 1, dans laquelle la composition a un glissement de 0,8 à 1,4 g selon un test de glissement sur une machine de test de distribution, de glissement et d'écaillage.

12. Composition selon la revendication 1, dans laquelle la composition a un écaillage inférieur à 25 %.
